# FASCICULE DE BREVET EUROPEEN

(11) **EP 0 506 550 B1**
(45) Date de publication et mention de la délivrance du brevet: **06.03.1996**
(21) Numéro de dépôt: 92400800.6
(22) Date de dépôt: 25.03.1992
(51) Int. Cl.: F16B 13/02, F16B 13/06, A61B 17/56

(54) **Cheville de fixation**
Befestigungsdübel
Fastening plug

(30) Priorité: 28.03.1991 FR 9103788
(43) Date de publication de la demande: 30.09.1992
(73) Titulaire: Fabre-Houviez, Claire, F-93310 Le Pré Saint Gervais (FR); Abadjian, Jean-Pierre, F-75004 Paris (FR); Beaumesnil, Jean-Claude, F-92400 Courbevoie (FR); Mory, Patrick, F-95120 Ermont (FR); Thierry, Michel, F-45100 Orléans (FR)
(72) Inventeur: Fabre-Houviez, Claire, F-93310 Le Pré Saint Gervais (FR); Abadjian, Jean-Pierre, F-75004 Paris (FR); Beaumesnil, Jean-Claude, F-92400 Courbevoie (FR); Mory, Patrick, F-95120 Ermont (FR); Thierry, Michel, F-45100 Orléans (FR)
(74) Mandataire: Casalonga, Axel

(56) Documents cités:
- WO-A-85/04221
- DE-B- 1 055 750
- FR-A- 788 263
- FR-A- 1 354 587

## Description

L'invention a pour objet une cheville de fixation.

On connaît des chevilles destinées à être fixées à des parois ou autre emplacement par insertion dans des trous préparés à cet effet, bloquées par insertion d'une vis, généralement d'une vis à bois.

Chacune de ces chevilles est destinée à une utilisation spécifique.

Ainsi, on utilise des chevilles de types différents pour des matériaux durs tels que le béton et des matériaux tendres tels que le plâtre. En outre, la taille des chevilles utilisées doit correspondre au diamètre et à la longueur des vis destinées à y être insérées. Ceci oblige à avoir en stock un grand nombre de chevilles.

WO-A-85/04221 enseigne une cheville cylindrique en forme d'hélice, obtenue par extrusion, dont la surface extérieure est lisse ou comporte des nervures transversales à l'axe de la cheville et dont l'intérieur est en forme d'étoile. Cette cheville ne comporte pas de stries longitudinales à sa surface extérieure, ni de bossage à sa surface intérieure.

FR-A-1.354.587 enseigne une cheville en matière plastique, obtenue par injection, comportant des stries extérieures et des cannelures internes. Cette cheville n'est pas découpée en forme d'hélice extensible mais se présente en une seule pièce. En outre, sa surface intérieure ne comporte pas de stries longitudinales et pas de bossages.

L'invention a pour objet une cheville qui surmonte les inconvénients des chevilles de l'état de la technique.

L'invention a pour objet une cheville de fixation multi-services, extensible, pouvant être découpée à la longueur désirée à partir d'une cheville mère de grande longueur et qui accepte des vis de diamètres différents.

L'invention a également pour objet une cheville pouvant être utilisée non seulement avec des vis à bois, mais également avec des vis à métaux.

L'invention a encore pour objet une cheville de fixation en forme d'hélice constituée de spires de forme cylindrique dont les deux surfaces, extérieure et intérieure de la cheville, comportent des stries longitudinales, et la surface intérieure comporte en outre un ou plusieurs bossage(s) constitué(s) par une surépaisseur d'une partie de la paroi intérieure de la cheville, cette surépaisseur formant bossage et qui est striée dans le sens longitudinal de la cheville représente de 5 à 60% du diamètre intérieur de la cheville et le ou les bossage(s) occupent de 5 à 50% de la circonférence intérieure de la paroi de la cheville.

Ladite cheville convient particulièrement bien pour être utilisée dans des matériaux durs, notamment le béton.

La cheville selon l'invention peut également être utilisée, après stérilisation, en chirurgie, notamment pour consolider des membres fracturés ou pour des prothèses.

Pour des matériaux tendres tels que le plâtre, ladite cheville est utilisée conjointement avec un manchon dans lequel est insérée la cheville.

Le manchon selon l'invention est de forme cylindrique. Il comporte à sa partie extérieure des saillies formant filetage et à une de ses extrémités, il comporte un épaulement.

Le filetage est progressif, la hauteur du filetage augmentant dans le sens de l'épaulement, le pas de vis étant maintenu constant.
La figure 1 représente une vue en élévation de l'extérieur de la cheville;
la figure 2 représente une vue en élévation de la cheville avec une vis partiellement insérée;
la figure 3 représente un agrandissement d'une coupe de la figure 1 selon III-III;
la figure 4 représente une coupe de l'ensemble manchon, cheville et vis.

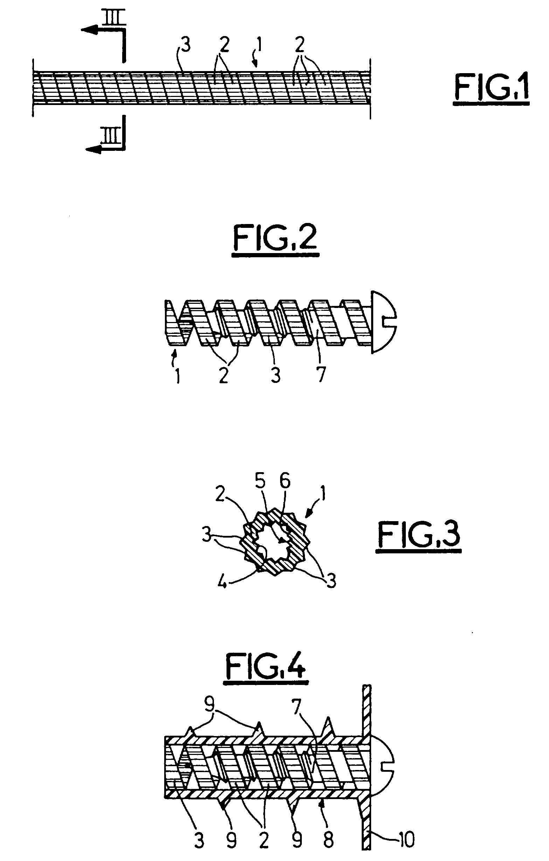

Sur la figure 1, on voit la cheville 1 formée de spires 2 portant des stries 3.

Sur la figure 2, on voit la cheville 1 dont les spires 2 portant les stries 3 s'écartent par suite de l'introduction de la vis 7.

Sur la figure 3, on voit les spires 2 portant les stries extérieures 3, les stries intérieures 4, et un bossage 5 portant des stries 6.

Sur la figure 4, on voit le manchon 8 avec le filetage 9 et l'épaulement 10, et à l'intérieur du manchon les spires 2 de la cheville dans laquelle s'insère la vis 7.

Le manchon 8 s'insère dans un matériau tendre sans détérioration de ce dernier grâce à son filetage progressif à pas de vis constant. L'épaulement limite la pénétration du manchon et sert d'appui au support.

Le filetage et l'épaulement du manchon assurent une bonne résistance au cisaillement et à l'arrachement.

Selon un mode de réalisation, l'extrémité terminale du manchon est entaillée sur une longueur de 5 mm au moins, afin de pouvoir s'écarter lors de l'introduction de la vis dans la cheville se trouvant à l'intérieur du manchon et augmenter la résistance à l'arrachement.

Selon un autre mode de réalisation, les deux extrémités du manchon sont entaillées sur 40 à 80% de la longueur du manchon.

Le plan de l'entaille de l'une des extrémités est perpendiculaire au plan de l'entaille de l'autre extrémité.

Les longueurs des entailles des deux extrémités peuvent être identiques ou différentes.

Ces entailles permettent l'écartement du manchon lors de l'introduction d'une vis dans la cheville et favorisent l'utilisation d'une vis ayant un diamètre supérieur au diamètre intérieur de la cheville. En outre, l'écartement du manchon améliore la résistance à l'arrachement.

Comme indiqué ci-dessus, la cheville porte à sa surface extérieure ainsi qu'à sa surface intérieure des stries longitudinales. Les stries extérieures permettent un bon accrochage de la cheville et évitent sa rotation dans le trou où elle est introduite. Les stries intérieures facilitent la pénétration de la vis.

L'intérieur de la cheville comporte un ou plusieurs bossages constitués par une surépaisseur d'une partie de la circonférence intérieure de la paroi de la cheville. La surépaisseur de la paroi intérieure formant bossage est de 5 à 60%, de préférence de 15 à 40% et plus préférablement de 10 à 35% du diamètre intérieur de la cheville. Le ou les bossages occupent de 5 à 50% et de préférence de 10 à 30% de la circonférence intérieure. Le ou les bossages peuvent porter des stries identiques ou similaires à celles se trouvant à la surface intérieure et extérieure de la cheville.

Grâce à son caractère extensible, la cheville en forme d'hélice peut être utilisée avec des vis ayant un diamètre plus grand que le diamètre intérieur de la cheville. Grâce à la présence du bossage, la cheville peut être utilisée avec des vis ayant un diamètre inférieur au diamètre intérieur de la cheville. En outre, la présence des stries à l'intérieur de la cheville ainsi que sur le ou les bossages, permet l'utilisation de vis à métaux qui, lors de leur pénétration à l'intérieur de la cheville, créent un filetage dans les stries, leur assurant un bon accrochage.

Lorsqu'on utilise des vis à bois, le diamètre des vis pouvant être insérées dans la cheville varie de 1 à 3 et de préférence de 1 à 4. Lorsqu'on utilise des vis à métaux, le diamètre des vis pouvant être insérées dans la cheville varie de 1 à 2, de préférence de 1 à 3.

La cheville et le manchon peuvent être fait en divers matériaux. La cheville est faite de préférence en matière synthétique et plus préférablement en polyamide rigide. Le manchon est fait de préférence en matière synthétique notamment en polyamide rigide ou en métal.

La matière synthétique telle que le polyamide, utilisée comme matière première pour la confection de la cheville et du manchon, est avantageusement additionnée de produits la rendant non inflammable et conforme à la norme américaine UL 94 et aux normes européennes, ce qui permet son utilisation dans des murs et portes coupe-feu et autres dispositifs formant coupe-feu ou pare-flammes.

Selon un mode de réalisation, chaque spire est chanfreinée sur tout son périmètre extérieur, sur un seul côté, dans le sens de pénétration de la cheville, dans un trou foré.

Le chanfrein a une inclinaison de 5 à 45° et de préférence de 20 à 45°. Les spires sont chanfreinées sur 5 à 25% de leur largeur.

Ce chanfrein assure une meilleure cohésion des spires entre elles, facilite l'introduction de la cheville dans le trou foré pour la recevoir. De plus, lorsque la vis est vissée à l'intérieur de la cheville, l'espace entre deux spires formée par le chanfrein se remplit de matériau, ce qui favorise un meilleur accrochage de la cheville aux parois du trou cylindrique où elle est placée et assure une plus grande résistance à l'arrachement.

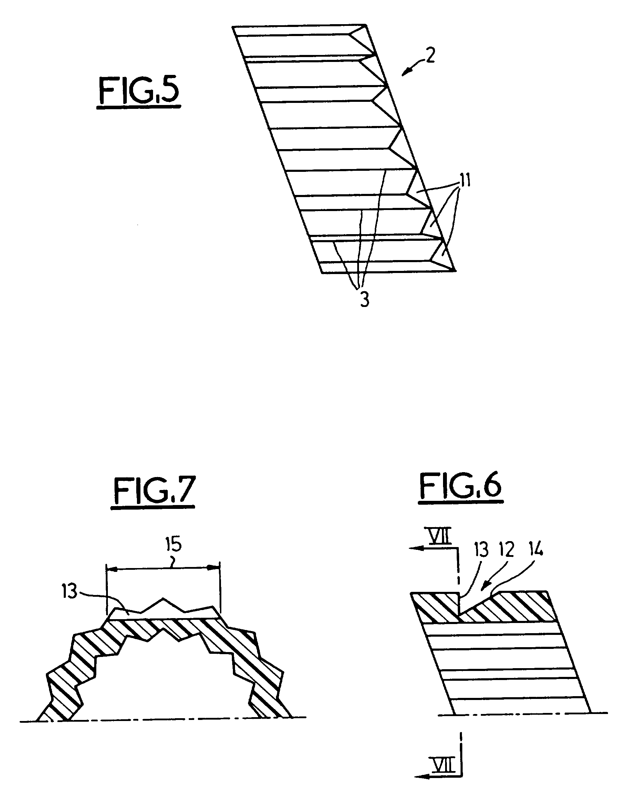

Le chanfrein est représenté sur la figure 5 qui reproduit une spire vue de face. Sur cette figure, on voit une spire 2 agrandie avec des stries 3, l'extrémité de la spire portant un chanfrein 11.

Selon un mode de réalisation, chaque spire de la cheville comporte une encoche 12 de profil triangulaire représentée sur les figures 6 et 7.

La figure 6 représente la section d'une spire, fortement agrandie, portant une encoche 12 et la figure 7 représente une coupe de la figure 6, selon VII-VII. Le côté 13 de l'encoche triangulaire 12, perpendiculaire à une génératrice de la cheville, correspond à la profondeur de l'encoche. Cette profondeur représente de 10 à 40% et de préférence de 15 à 30% de l'épaisseur de la spire.

Le côté 14 de l'encoche triangulaire forme avec le côté 13 un angle de 30° à 60° et avantageusement de 40 à 50°.

Sur la figure 7, on voit la profondeur 13 et la longueur 15 de l'encoche. La longueur de l'encoche est de 1 à 4 mm. L'encoche coupe de 1 à 4 stries.

Chaque spire peut comporter une ou plus d'une encoche. Lorsqu'une spire comporte une seule encoche, les encoches pratiquées sur les spires successives peuvent former une ligne droite ou bien être décalées les unes par rapport aux autres. Les encoches des spires successives peuvent former avantageusement une hélice.

Lorsque chaque spire comporte deux encoches, elles peuvent également former des lignes droites ou des hélices ou être autrement décalées les unes par rapport aux autres.

La présence des encoches favorise l'accrochage de la cheville aux parois de trou foré dans le matériau et plus particulièrement dans les matériaux granuleux. Lors de l'introduction de la vis dans la cheville, les encoches se remplissent de matériau, ce qui augmente la résistance à l'arrachement de la cheville.

Normalement, les stries des différentes spires forment une nervure longitudinale qui est parallèle à une génératrice du cylindre.

Selon une variante, l'hélice qui constitue la cheville comporte une torsion supplémentaire qui a pour résultat que la nervure formée par les stries est inclinée par rapport à une génératrice et forme également une hélice. Cette inclinaison est de 10 à 20° par rapport à une génératrice.

L'invention sera mieux comprise à l'aide des exemples non limitatifs suivants.

### EXEMPLE 1

On fabrique par extrusion à partir d'un polyamide d'une dureté shore de 60, une cheville en forme d'hélice, striée à l'extérieure et à l'intérieure et munie en outre à l'intérieur d'une surépaisseur de 0,6 mm formant bossage. Ce bossage occupe 16,6% de la circonférence intérieure de la cheville. Le bossage comporte également des stries longitudinales.

L'hélice cylindrique a un diamètre extérieur de 7 mm et un diamètre intérieur de 4,5 mm.

On fabrique également un manchon à partir d'un polyamide d'une dureté de 160 shore.

Cette cheville peut être utilisée dans des matériaux durs avec des vis en bois d'un diamètre de 2,5 à 10 mm et avec des vis à métaux d'un diamètre de 2,5 à 8 mm.

Le manchon a un diamètre extérieur de 10,5 mm, un diamètre intérieur de 7,5 mm.

### EXEMPLE 2

On fabrique par extrusion à partir d'un polyamide de dureté shore 60, une cheville en forme d'hélice, striée longitudinalement à l'extérieur et à l'intérieur et comportant à l'intérieur une surépaisseur de 1 mm formant bossage. Ce bossage occupe 16,6% de la circonférence intérieure de la cheville. Les spires ont un diamètre intérieur de 4,5 mm, une épaisseur de 2,25 mm et un diamètre extérieur de 9 mm. Chaque spire comporte une encoche ayant une profondeur de 0,7 mm et une longueur de 2 mm. Les encoches des spires successives sont décalées les unes par rapport aux autres de façon à former une hélice.

### EXEMPLE 3

On fabrique par extrusion une cheville identique à celle de l'exemple 2, avec la différence que chaque spire est chanfreinée sur une de ses extrémités sur 10% de sa largeur d'un chanfrein ayant une inclinaison de 40° par rapport à une génératrice de la cheville.

## Revendications

1. Cheville de fixation (1) extensible, en forme d'hélice, constituée de spires (2) de forme cylindrique, dont la surface extérieure n'est pas lisse et dont la surface intérieure comporte des stries longitudinales (4), caractérisée en ce que les deux surfaces, extérieure et intérieure de la cheville, comportent des stries longitudinales (3), (4) et que la surface intérieure comporte en outre un ou plusieurs bossage(s) (5) constitué(s) par une surépaisseur d'une partie de la paroi intérieure de la cheville; cette surépaisseur formant bossage et qui est également striée dans le sens longitudinal de la cheville, représente de 5% à 60% du diamètre intérieur de la cheville et le ou les bossage(s) occupent de 5 à 50% de la circonférence intérieure de la paroi de la cheville.

2. Cheville selon la revendication 1, caractérisée en ce qu'elle est constituée de deux parties, une première partie comprend la cheville (1) proprement dite telle que définie dans la revendication 1, la deuxième partie est constituée par un manchon (8) destiné à entourer la cheville proprement dite, ce manchon de forme cylindrique comporte à sa partie extérieure des saillies formant filetage (9) ainsi qu'un épaulement (10).

3. Cheville selon la revendication 1, caractérisée en ce que le ou les bossages (5) occupent de 10 à 30% de la circonférence intérieure.

4. Cheville selon la revendication 1, caractérisée en ce que la surépaisseur de la paroi intérieure formant bossage est de 15 à 40% du diamètre intérieur de la cheville.

5. Cheville selon la revendication 2, caractérisée en ce que les deux extrémités du manchon sont entaillées sur 40 à 80% de la longueur du manchon, le plan de l'entaille de l'une des extrémités est perpendiculaire au plan de l'entaille de l'autre extrémité, les longueurs des entailles des deux extrémités pouvant être identiques ou différentes.

6. Cheville selon les revendications 1 à 4, caractérisée en ce qu'elle se présente sous la forme d'une cheville mère fabriquée par extrusion, pouvant être découpée en plusieurs chevilles ayant la longueur désirée.

7. Cheville selon les revendications 1 à 4 et 6, caractérisée en ce que chaque spire est chanfreinée sur tout son périmètre extérieur, sur un seul côté.

8. Cheville selon les revendications 1 à 7, caractérisée en ce que chaque spire de la cheville comporte une encoche de profil triangulaire ayant une profondeur égale à 10 à 40% de l'épaisseur d'une spire.

9. Cheville selon la revendication 8, caractérisée en ce que la longueur de l'encoche est de 1 à 4 mm.

10. Cheville selon les revendications 8 et 9, caractérisée en ce que chaque spire comporte une ou plus d'une encoche.

11. Cheville selon la revendication 10, caractérisée en ce qu'elle comporte une seule encoche et les encoches pratiquées sur les spires successives sont décalées les unes par rapport aux autres.

12. Cheville selon la revendication 11, caractérisée en ce que les encoches pratiquées sur les spires successives forment une hélice.

13. Cheville selon les revendications 1 à 12, caractérisée en ce que la nervure formée par les stries est inclinée par rapport à une génératrice de 10 à 20°.

14. Cheville selon les revendications 1, 3, 4 et 6 à 13, destinée à être utilisée en chirurgie, pour consolider des membres fracturés ou pour des prothèses.

## Patentansprüche

1. Dehnbarer Befestigungsdübel (1) in Schraubenform, bestehend aus Schraubengängen (2) zylindrischer Form, dessen äußere Oberfläche nicht glatt ist und dessen innere Oberfläche Längsrillen (4) aufweist, dadurch gekennzeichnet, daß die beiden Oberflächen, die äußere und die innere, des Dübels Längsrillen (3), (4) aufweisen und die innere Oberfläche ferner einen Wulst oder mehrere Wülste (5) aufweist, die aus einer Überdicke eines Teils der Innenwand des Dübels bestehen; wobei diese den Wulst bildende Überdicke, der ebenfalls in Längsrichtung des Dübels gerillt ist, 5% bis 60% des Innendurchmessers des Dübels darstellt und der Wulst oder die Wülste 5 bis 50% des Innenumfangs der Dübelwand in Anspruch nehmen.

2. Dübel gemäß Anspruch 1, dadurch gekennzeichnet, daß dieser aus zwei Teilen besteht, wobei der erste Teil den eigentlichen Dübel (1) umfaßt, wie er in Anspruch 1 definiert ist, und der zweite Teil aus einer Hülse (8) besteht, die dazu bestimmt ist, den eigentlichen Dübel zu umgeben, wobei diese Hülse in Zylinderform an ihrem Außenteil Vorsprünge, die ein Außengewinde (9) bilden, sowie einen Bund (10) aufweist.

3. Dübel gemäß Anspruch 1, dadurch gekennzeichnet, daß der Wulst oder die Wülste (5) 10 bis 30% des Innenumfangs einnehmen.

4. Dübel gemäß Anspruch 1, dadurch gekennzeichnet, daß die Überdicke der den Wulst bildenden Innenwand 15 bis 40% des Innendurchmessers des Dübels beträgt.

5. Dübel gemäß Anspruch 2, dadurch gekennzeichnet, daß die zwei Enden der Hülse über 40 bis 80% der Länge der Hülse eingekerbt sind, wobei die Ebene der Einkerbung des einen Endes senkrecht auf der Ebene der Einkerbung des anderen Endes steht und die Längen der Einkerbungen der zwei Enden identisch oder unterschiedlich sein können.

6. Dübel gemäß den Ansprüchen 1 bis 4, dadurch gekennzeichnet, daß sich der Dübel als Einheitsform darstellt, die durch Strangpressen gefertigt wird und in eine Reihe von Dübeln in gewünschter Länge abgeschnitten werden kann.

7. Dübel gemäß den Ansprüchen 1 bis 4 sowie 6, dadurch gekennzeichnet, daß jeder Schraubengang über seinen gesamten Außenumfang auf nur einer Seite gefast ist.

8. Dübel gemäß den Ansprüchen 1 bis 7, dadurch gekennzeichnet, daß jeder Schraubengang des Dübels eine Einkerbung mit dreieckigem Profil aufweist, die eine Tiefe von 10 bis 40% der Dicke eines Schraubengangs hat.

9. Dübel gemäß Anspruch 8, dadurch gekennzeichnet, daß die Länge einer Einkerbung 1 bis 4 mm beträgt.

10. Dübel gemäß den Ansprüchen 8 und 9, dadurch gekennzeichnet, daß jeder Schraubengang eine oder auch mehr als eine Einkerbung aufweist.

11. Dübel gemäß Anspruch 10, dadurch gekennzeichnet, daß er nur eine Einkerbung aufweist, und die in den nachfolgenden Schraubengängen eingebrachten Einkerbungen gegeneinander versetzt angeordnet sind.

12. Dübel gemäß Anspruch 11, dadurch gekennzeichnet, daß die in den nachfolgenden Schraubengängen eingebrachten Einkerbungen eine Schraubenlinie bilden.

13. Dübel gemäß den Ansprüchen 1 bis 12, dadurch gekennzeichnet, daß die durch die Rillen gebildete Rippe gegenüber einer Erzeugenden um 10 bis 20° geneigt ist.

14. Dübel gemäß den Ansprüchen 1, 3, 4 und 6 bis 13, der zur Anwendung in der Chirurgie bestimmt ist, um gebrochene Glieder oder Prothesen zu befestigen.

## Claims

1. A helix-shaped extensible fixing dowel (1) consisting of cylindrical-shaped turns (2) whose outer surface is not smooth and whose inner surface comprises longitudinal ribs (4), characterized in that both outer and inner surfaces comprises longitudinal ribs (3), (4) and in that the inner surface also comprises one or more bulge(s) (5) constituted by an extra thickness of a portion of the inner wall of the dowel, this bulge-forming extrathickness and which is also ribbed in the longitudinal direction of the dowel, corresponding from 5% to 60% of the inner diameter of the dowel and this or these bulges occupying 5% to 50% of the inner circumference of the dowel wall.

2. Dowel as claimed in claim 1, characterized in that it consists of two portions, a first portion comprises the actual dowel (1) such as defined in claim 1, the second portion is constituted by a sleeve (8) intended to surround the actual dowel, this cylindrical-shaped sleeve comprises thread-forming projections (9) on its outer portion and a shoulder (10).

3. Dowel as claimed in claim 1, characterized in that the bulge or bulges occupy from 10 to 30 % of the inner circumference.

4. Dowel as claimed in claim 1, characterized in that the bulge-forming extra thickness of the inner wall is from 15 to 40 % of the inner diameter of the dowel.

5. Dowel as claimed in claim 2, characterized in that the two ends of the sleeve are slitted over 40 to 80 % of the length of the sleeve, the plane of the slit of one of the ends is perpendicular to the plane of the slit of the other end, the lengths of the slits of the two ends being able to be identical or different.

6. Dowel as claimed in claim 1 to 4, characterized in that it has the shape of an extruded mother dowel from which several dowels may be cut to the desired length.

7. Dowel as claimed in claims 1 to 4 and 6 characterized in that each turn is chamfered over its entire outer perimeter, on a single side.

8. Dowel as claimed in claims 1 to 7 characterized in that each turn of the dowel comprises a triangular-profile notch having a depth equal to 10 to 40 % of the thickness of a turn.

9. Dowel as claimed in claim 8, characterized in that the length of the notch is from 1 to 4 mm.

10. Dowel as claimed in claims 8 and 9 characterized in that each turn comprises one or more than one notch.

11. Dowel as claimed in claim 10, characterized in that it comprises one notch and the notches made on successive turns are offset with respect to one another.

12. Dowel as claimed in claim 11, characterized in that the notches made on successive turns form a helix.

13. Dowel as claimed in claim 1 to 12, characterized in that the ribbing formed by the ribs is inclined in relation to a generatrix by 10 to 20°.

14. Dowel as claimed in claim 1, 3, 4 and 6 to 13 intended to be used in surgery, for setting fractured limbs or for prostheses.
